# EUROPEAN PATENT SPECIFICATION

(11) **EP 4 408 449 B1**
(45) Date of publication and mention of the grant of the patent: **26.03.2025**
(21) Application number: 23706726.9
(22) Date of filing: 20.02.2023
(51) Int. Cl.: A61K 36/074, A61K 36/185, A61K 31/01, A61K 33/04, A61K 31/4375, A61K 36/47, A61K 36/29, A61K 36/42, A61P 35/00

(54) **HERBAL COMPOSITION FOR PROSTATE HEALTH AND PROSTATE CANCER PREVENTION**
PFLANZLICHE ZUSAMMENSETZUNG ZUR PRÄVENTION VON PROSTATAGESUNDHEIT UND PROSTATAKREBS
COMPOSITION À BASE DE PLANTES POUR LA SANTÉ DE LA PROSTATE ET LA PRÉVENTION DU CANCER DE LA PROSTATE

(30) Priority: 23.02.2022 US 202263313018 P
(43) Date of publication of application: 07.08.2024
(73) Proprietor: Mann Kevehazi, Laura, 8509 Kouklia (CY)
(72) Inventor: Mann Kevehazi, Laura, 8509 Kouklia (CY)
(74) Representative: Mewburn Ellis LLP
(86) International application number: PCT/EP2023/054192
(87) International publication number: WO 2023/161184

(56) References cited:
- WO-A1-2004/064814
- TW-U- M 604 565
- US-A1- 2008 260 771
- US-A1- 2010 034 762
- US-A1- 2013 115 202
- LI C ET AL: "Pharmaceutical composition in preparing medicine for treating prostatic hyperplasia, comprises African pygeum bark extract, nettle root extract, beta-sitosterol, lycopene, zinc citrate, selenium and vitamin E", WPI,, vol. 2020, no. 33, 24 March 2020 (2020-03-24), XP002808832
- ZHENG H: "Maca powder containing product e.g. for enhancing male and female sexual desire, improving sperm quality and quantity of sperm, improving erectile dysfunction, and preventing addition to effect of enlarged treating prostate and protecting prostate tissue", WPI,, vol. 2021, no. 56, 1 December 2020 (2020-12-01), XP002808831
- DANIEL SLIVA: "Suppression of growth and invasive behavior of human prostate cancer cells by ProstaCaidTM: Mechanism of activity", INTERNATIONAL JOURNAL OF ONCOLOGY, 4 April 2011 (2011-04-04), GR, XP055429107, ISSN: 1019-6439, DOI: 10.3892/ijo.2011.996
- DATABASE WPI Week 2021005, Derwent World Patents Index; AN 2021-742129, XP002808831
- DATABASE WPI Week 2020003, Derwent World Patents Index; AN 2020-242632, XP002808832

## Description

### TECHNICAL FIELD

The present invention relates to compositions which can be used to protect the prostate tissue and inhibit prostate cancer development, as well as mitigating urinary tract symptoms caused by prostate enlargement. More particularly, the composition comprises multiple botanical/herbal extracts and mineral ingredients protect the prostate tissue and inhibit prostate cancer development.

### BACKGROUND

The prostate gland is intimately involved in the health of any man. With age Benign Prostate Hyperplasia (BPH) becomes the most common problem impacting men's lives for decades. According to the American Urology Association, half of men between ages 51-60 have documented Benign Prostate Hyperplasia (BPH) and close to 90% suffer from Lower Urinary Tract Symptoms (LUTS).

Prostate enlargement is a long term condition fuelled by chronic inflammation at cellular level. The link between chronic inflammation and carcinogenesis was proven in multiple studies. Research at John Hopkins School of Medicine has shown that in spite of low PSA levels, chronic inflammation in the prostate more than doubles the risk of aggressive prostate cancer.

Prostate cancer affects one in six Caucasian men and a staggering one in four men of African ethnic background.

Fifty years ago, President Richard Nixon declared 'war on cancer' and signed it into law hoping to find a cure. Yet, in spite of the huge amounts of funding and research invested in finding a cure, fifty years down the line, in spite of extensive national screening, cancer continues to be a life altering diagnostic affecting 1 in 6 or 1 in 4 men and their families.

The current screening and diagnostic procedures for prostate cancer are invasive scans (Transrectal Ultrasound, Transrectal Prostate Biopsy) with a high risk of infection, can lead to a high amount of false negatives or false positives, as well as being expensive, and very unpleasant for the patient.

The cancer treatments include transurethral Prostatectomy, radiation, and chemotherapy with a high risk for infection and serious side effects: impotence, urethral scarring, urinary incontinence, chronic urinary tract infections, urinary retention that require catheterization, and sepsis by way of non limiting example. These are a heavy burden on the healthcare system and a huge toll on the patients mental health and their families.

Currently, there are no safe and effective ways to prevent prostate carcinogenesis at cellular level before a tumor is formed.

There is an urgent need to reduce the high rates of prostate malignancy affecting one in six Caucasian men and one in four men of African ethnicity. At the same time there is a need for a safe and affordable solution for mitigating the urinary symptoms caused by Benign Prostate Hyperplasia that affect the overwhelming majority of men over the age of 50.

The present invention has been devised in light of the above considerations.

Jiang J et al. in "Suppression of growth and invasive behavior of human prostate cancer cells by ProstaCaid™: mechanism of activity" (Int J Oncol. 2011;38(6):1675-82. doi: 10.3892/ijo.2011.996) describe using ProstaCaid to inhibit invasive human prostate cancer, ProstaCaid including, *inter alia,* extracts from medicinal mushrooms, pumpkin seed, berberine, selenium, and lycopene.

US20100034762A1 describes preparing extracts of Embilica officinalis for use in medicaments, for example for the treatment of prostatic cancer.

WO2004064814 describes a composition including, *inter alia,* nettle root extract (Urtica doica) 16:1 and pumpkin seed oil (Cucubita pepo), for example for use in assisting the treatment of benign prostate hypertrophy.

US20080260771A1 describes a composition for the prevention and treatment of prostate disorders, the composition including, *inter alia,* Ganoderma lucidum, Selenium, Cucurbita pepo, and Urtica doica.

TWM604565U describes powders for enhancing male and female sexual desire, improving sperm quality, improving erectile dysfunction, and protecting prostate tissue, the powder including maca powder and pumpkin seed powder.

US20130115202 describes methods of treating neuro-inflammation disorders with a composition including, *inter alia,* berberine and optionally selenium.

CN110898114A describes a pharmaceutical composition for use in preparing medicine for treating prostatic hyperplasia, the composition including, *inter alia,* nettle root extract, lycopene, and selenium.

### BRIEF DESCRIPTION OF THE DRAWINGS

The present disclosure will be better understood by reading the written description with reference to the accompanying drawing figures in which like references numerals denote similar structure and referred to like elements throughout which:
Figure 1 is a graphical representation of the efficacy of the formula used in accordance with the invention.
Figure 2 is field images of PC-3 cells, before application of current tested composition (non-treated) as well as 24 and 48 hours after the application.
Figure 3 is a graphical representation of the efficacy of the composition -specifically the inhibitory effect on the viability of the Prostate cancer cells used in accordance with the invention, as well as the synergistic effect of the composition compared to the individual ingredients (Pumpkin seed extract, Nettle root, Reishi mushroom, Amla berry and Berberine alone) and a control.

### SUMMARY OF THE INVENTION

A first aspect of the present invention provides a composition comprising Pumpkin seed extract; Nettle root extract (Urtica dioica); Berberine; Emblica officinalis (Amla berry); Ganoderma Lucidum (Reishi mushroom); Lycopene; and Selenium.

More particularly in one embodiment the composition is in the form of tablets or capsules and comprises Pumpkin seed extract to from 500-1000mg per daily dose; Nettle root extract (Urtica dioica) to between 300-700mg per daily dose; Berberine Hydrochloride 90% from 50-100mg per daily dose; Emblica officinalis (Amla berry) from 50- 100mg per daily dose; Ganoderma Lucidum (Reishi mushroom) from 50-100mg per daily dose; Lycopene from 5-10mg per daily dose; and Selenium in the form of L-Selenomethionine, Sodium selenite or Sodium selenate from 30-40mcg per daily dose. The recommended daily dose amount can be reached by a single dosage form such as a liquid or gel, or by several tablets or capsules.

In another embodiment of the present invention the composition comprises: Pumpkin seed extract equivalent to between 1000-4000mg Pumpkin seed per dose; Nettle root extract (Urtica dioica) equivalent to between 500- 2000mg Nettle root per dose; Berberine Hydrochloride 90% 50-300mg per dose; Emblica officinalis (Amla berry) 50-300mg per dose; Ganoderma Lucidum (Reishi mushroom) 50- 300mg per dose; Lycopene 5-20mg per dose; and Selenium from 40-160mcg per dose. In a further aspect the composition is in the form of a powder. In another aspect the composition is in the form of a liquid or a gel.

In another aspect of the invention the composition is in the form of a liquid, gel or powder comprising: 400mg of Pumpkin seed extract 10:1 equivalent of 4000mg Pumpkin seed; 200mg of Nettle root extract 10:1 (Urtica dioica) doica) equivalent to 2000mg Nettle root; 200-300mg of Berberine Hydrochloride; 200-300mg of Emblica Officinalis (Amla berry); 200-300mg of Ganoderma lucidum (Reishi mushroom); 20mg of Lycopene; and 160mcg of Selenium. In some embodiments the liquid, gel or powder is administered as a single daily dose.

In another embodiment of the present invention the composition is in the form of a tablet or a capsule comprising: Pumpkin seed extract equivalent to 1000-2000mg Pumpkin seed; Nettle root extract (Urtica dioica) doica) equivalent to 500-1000mg Nettle root; 50-150mg of Berberine Hydrochloride 90%; 50-150mg of Emblica officinalis (Amla berry); 50-150mg of Ganoderma Lucidum (Reishi mushroom); 5-10mg of Lycopene; and 40-80mcg of Selenium. In further embodiments the composition is in the form of a tablet or a capsule comprising: Pumpkin seed extract equivalent to 1000mg of Pumpkin seed; Nettle root extract (Urtica dioica) doica) equivalent to 500mg of Nettle root; 50mg of Berberine Hydrochloride 90%; 50mg of Emblica officinalis (Amla berry); 50mg of Ganoderma Lucidum (Reishi mushroom) per dose; 5mg of Lycopene; and 40mcg of Selenium. In some embodiments 2-4 tablets/capsules can be administered per day.

In yet another embodiment of the present invention the composition is in the form of a liquid or gel having Pumpkin seed extract equivalent to about 4000mg Pumpkin seed per daily dose; Nettle root extract (Urtica dioica) equivalent to about 2000mg Nettle root per daily dose; Berberine Hydrochloride 90% of about 200mg per daily dose; Emblica officinalis (Amla berry) of about 200mg per daily dose; Ganoderma Lucidum (Reishi mushroom) of about 200mg per daily dose; Lycopene 15of about 20mg per daily dose; and Selenium of about 160mcg per daily dose.

In still another embodiment of the present invention the composition is in the form of a tablet having Pumpkin seed extract between 1000-4000mg per daily dose; Nettle root extract (Urtica doica) between 500-2000mg per daily dose; Berberine Hydrochloride 90% between 50-200mg per daily dose; Embilica officinalis (Amla berry) between 50-200mg per daily dose; Ganoderma Lucidum (Reishi mushroom) between 50-200mg per daily dose; Lycopene between 5-20mg per daily dose; and Selenium from 4-160mcg per daily dose. In some embodiments 2-4 tablets can be administered per day.

In some embodiments, the compositions of the present invention comprise pharmaceutically acceptable excipients. Excipients for different embodiments, capsules, soft capsules, gel, powder sachet or liquid may include, as illustrative examples, one or more of: gelling agent(s), surfactant(s), emulsifier(s), solubilizer(s), surfactant(s) (nonionic), plasticizer(s), matrix forming agent(s), sweetener(s), bulking/diluent agent(s), preservative(s), anti-caking/lubricant(s), coating agent(s). Illustrative embodiments such excipients may include, PEG-35 Castor Oil, Polysorbate 80, Poloxamers, Polyethyleneglycol-15 -hydroxystearate, Stevia (sweetener), microcrystalline cellulose (bulking/diluent agents), or silicon dioxide (anti caking/lubricant agents). The above listed compounds are illustrative only. Other excipients known to one of skill in the art may be used in addition to, or alternative to. In further embodiments, the composition may comprise one or more of pharmaceutically acceptable diluent(s), lubricant(s), glazing and coating agent(s).

In another aspect of the invention the compositions are for preventing prostate cancer, protecting the prostate cells from malignant changes and significantly lowering the risk of prostate cancer.

In another embodiment the compositions are for use in mitigating the urinary symptoms of an enlarged prostate. In a further embodiment the compositions are for use in treating urinary symptoms triggered by an enlarged prostate optionally wherein the urinary symptoms are selected from frequency, nocturia, straining and urgency of urination. In another embodiment the compositions are for use in treating erectile dysfunction or improving sexual function.

Also disclosed is a method of preventing prostate cancer and protecting the prostate cells from malignant changes may comprise, administering to an individual in need thereof, any composition (in any concentrations or dosages) as described or contemplated herein ( i.e., comprising Pumpkin seed extract; Nettle root extract (Urtica dioica); Berberine Hydrochloride; Emblica officinalis (Amla berry); Ganoderma Lucidum (Reishi mushroom); Lycopene; and Selenium; and optionally one or more of pharmaceutically acceptable excipients or diluents, lubricants, glazing and coating agents.

Also disclosed is a method of lowering or reducing the risk of prostate cancer and protecting the prostate cells from malignant changes may comprise, administering to an individual in need thereof, any composition (in any concentrations or dosages) as described or contemplated herein ( i.e., comprising Pumpkin seed extract; Nettle root extract (Urtica dioica); Berberine Hydrochloride; Emblica officinalis (Amla berry); Ganoderma Lucidum (Reishi mushroom); Lycopene; and Selenium; and optionally one or more of pharmaceutically acceptable excipients or diluents, lubricants, glazing and coating agents.

Also disclosed is a method of mitigating the urinary symptoms of an enlarged prostate may comprise, administering to an individual in need thereof, any composition (in any concentrations or dosages) as described or contemplated herein ( i.e., comprising Pumpkin seed extract; Nettle root extract (Urtica dioica); Berberine Hydrochloride; Emblica officinalis (Amla berry); Ganoderma Lucidum (Reishi mushroom); Lycopene; and Selenium; and optionally one or more of pharmaceutically acceptable excipients or diluents, lubricants, glazing and coating agents.

Also disclosed is a method of treating urinary symptoms triggered by an enlarged prostate may comprise, administering to an individual in need thereof, any composition (in any concentrations or dosages ) as described or contemplated herein (i.e., comprising Pumpkin seed extract; Nettle root extract (Urtica dioica); Berberine Hydrochloride; Emblica officinalis (Amla berry); Ganoderma Lucidum (Reishi mushroom); Lycopene; and Selenium; and optionally one or more of pharmaceutically acceptable excipients or diluents, lubricants, glazing and coating agents.

Also disclosed is a method of treating urinary symptoms triggered by an enlarged prostate, optionally wherein the urinary symptoms are selected from frequency, nocturia, straining and urgency of urination, may comprise, administering to an individual in need thereof, any composition (in any concentrations or dosages) as described or contemplated herein (i.e., comprising Pumpkin seed extract; Nettle root extract (Urtica dioica); Berberine Hydrochloride;

Emblica officinalis (Amla berry); Ganoderma Lucidum (Reishi mushroom); Lycopene; and Selenium; and optionally one or more of pharmaceutically acceptable excipients, or diluents, lubricants, glazing and coating agents.

The invention includes the combination of the aspects and preferred features described except where such a combination is clearly impermissible or expressly avoided.

### DETAILED DESCRIPTION

Aspects and embodiments of the present invention will now be discussed. Further aspects and embodiments will be apparent to those skilled in the art.

Modern lifestyles involve exposure to a variety of carcinogenic factors: smoking, chemicals, outdoor or indoor pollution, radiation or toxins which generate tiny particles called free radicals. Free radicals can damage all major components of cells, including DNA, proteins, and cell membranes leading to oxidative stress, chronic inflammation and cellular disfunction setting up the process of carcinogenesis.

Cancer is a complex disease driven by chronic inflammation through metabolic pathways, proven to actively contribute to cancer initiation, promotion and progression. Prostate cancer as used herein is a generic term for tumors of the prostate as each tumor consists of genetically different diverse cancer cells and targeting just one or two processes leads to rapid additional mutations evading the targeted therapies.

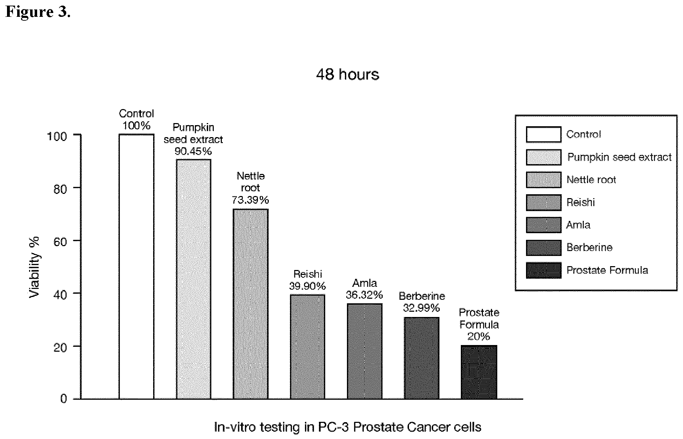

As a result, no single solution or approach to date provides sustainable result providing both cellular protection and improved bladder and urinary comfort. Using just one ingredient can have side effects and will not comprehensively address all the mechanisms through which cancer takes hold in our cells, develops and spreads. The above ingredients synergistically provide the desired benefits; more than each one individually. This synergistic effect of the composition compared to separate ingredients is demonstrated by Example 1 and Figure 3.

This is the precise reason why the present composition includes several ingredients, each addressing several aspects of cancer cell metabolism: antioxidant activity, anti-inflammatory activity, modulation of inflammatory enzymes, immune response, inhibition of rapid multiplication by inducing apoptosis, inhibition of angiogenesis, inhibition of enzymes that enable spread into the extracellular matrix, and activation of detoxifying mechanisms.

The combination of the various phytochemicals and minerals in lower doses avoids the possible side effects of several ingredients providing synergistic effects with each other while avoiding toxicity. The potential exists for the current herbal and mineral composition to provide cellular antioxidant protection, as well as prophylactic and potentially chemo preventive effect with daily administration.

Daily amounts of phytochemicals, antioxidants and minerals as described below, targeted for cellular prostate health, effectively neutralize free radicals, mitigate chronic inflammatory pathways, prevent and combat cellular damage, representing a natural, nontoxic, and affordable long-term chemoprevention. In this composition low amounts of each ingredient are included in order to provide a desired chemo-preventive fighting effect while also mitigating the chronic inflammation present in BPH and its effects on the bladder function while avoiding any possible side effects.

The proposed formulations include the following ingredients:

**EMBLICA OFFICINALIS (Amla Berry)** at 260,000 ORAC units, the amla berry has the highest antioxidant capacity (free radical scavenging capacity) of all fruits and vegetables; that is 27 times more than wild blueberries at 9,600 ORAC units.

Amla's high level of antioxidant capacity may be due to high content of content of vitamin C, polyphenols, such as flavonoids and hydrolysable tannins, that exert its strong anti-inflammatory activity, modulation of various enzymes of inflammation like NF-kB which is one of the main drivers of carcinogenesis leading to apoptosis in neoplastic cells.

The amount of Emblica officinalis in the composition is in the range of 50-300mg per dose. In further embodiments the amount of Emblica Emblica officinalis in the composition is in the range of 50-200mg per dose. The daily dose of Emblica officinalis is 200-300mg.

**GANODERMA LUCIDUM (Reishi mushroom)** contains beta-glucan polysaccharides and triterpenoids. The chemo preventive activity of Reishi is exerted through multiple mechanisms: downregulation of estrogen - alpha receptors and NF-kB pathway, antiangiogenic effect through down regulation of VGEF, inhibition of metalloproteinase (MMP-9) downregulates cellular migration and induction of apoptosis in cancer cells.

Prostate tissue has an abundance of Estrogen receptors, both ER-alpha and ER- beta that drive carcinogenesis, and upregulate tumor development.

Ganoderma down regulates the Estrogen -alpha receptors found to mediate high grade prostatic intraepithelial neoplasia. The Ganoderma Lucidum can be in the form of the fruiting body of the mushroom, which is the stalk and cap, and comprises the organism's reproductive structure. The amount of Ganoderma in the composition is in the range of 50-300mg per dose. In further embodiments the amount of Ganoderma lucidum in the composition is in the range of 50-200mg per dose. The daily dose of Ganoderma lucidum is 200-300mg.

**BERBERINE** - Berberine is an isoquinoline plant alkaloid with the chemical formula C₂₀H₁₈CINO₄, widely present in different traditional medicines. In the present composition an orally available synthetic form of Berberine was used which is Berberine Hydrochloride with the chemical formula C₂₀H₂₂CINO₆. It was used in the present composition for its direct effect on prostatic stromal and glandular components, known to be involved in the development of BPH. Berberine acts on the prostate cells through inhibiting the inflammatory microenvironment at cellular level, interfering with the metabolic changes leading to cancer initiation, inhibiting carcinogenesis and inducing prostate cancer cells apoptosis. Berberine in high amounts can cause altered liver function, gastric symptoms, hepato and hematotoxicity, hemorrhagic inflammatory consequences, damage to immune cells. Therefore the amount of Berberine in the composition is in the range of 50-300mg per dose. In further embodiments the amount of Berberine in the composition is in the range of 50-200mg per dose. The daily dose of Berberine is 200-300mg. In some embodiments the Berberine is in the synthetic form Berberine Hydrochloride (C₂₀H₂₂CINO₆).

**PUMPKIN SEED EXTRACT** - has an antioxidant and anti-inflammatory effect while having a dual beneficial effect on the bladder and prostate. It directly contributes to improvement in the urinary symptoms caused by BPH: frequency, urgency, nocturia, weak stream and straining, thus greatly improving the quality of life of men affected by BPH.

Pumpkin seed extract is used in amounts of 500- 4000mg per daily dose. The pumpkin seed extract can be water soluble. The Pumpkin seed extract used can be Pumpkin seed extract 10:1. Pumpkin seed extract 10:1 (1 mg extract is the equivalent to 10mg of pumpkin seed) means the daily dose equivalent to 4000mg is reached by 400mg of extract. In further embodiments the amount of pumpkin seed extract in the composition is in an amount equivalent to 1000-4000mg Pumpkin seed per dose. The daily dose of Pumpkin seed extract is equivalent to 4000mg Pumpkin seed.

**NETTLE ROOT EXTRACT** - (Urtica dioica) contains multiple antioxidant and anti-inflammatory molecules (quercetin, rutin, kampfaerol) and it is used in the current composition for its effects on mitigating prostate enlargement; Benign Prostate Hyperplasia (BPH). Stinging nettle blocks the oxidation of fats thereby protecting many tissues from oxidative stress, as well as reducing inflammatory cytokine release and inflammatory biomarkers like TF-kB and interleukins, thus exerting an antiproliferative effect on prostate cells.

Nettle root extract increases the effect of blood pressure medication as well as diabetes medication, hence using it in the correct amounts can simultaneously provide a chemopreventive effect against other medications as well as mitigate the effects of an enlarged prostate, have a beneficial effect on the bladder that is inevitably negatively impacted by an enlarged prostate. The Nettle root extract used can be Nettle root extract 10:1. For example Nettle root extract 10:1 (1 mg extract is the equivalent to 10mg of Nettle root) means the daily dose equivalent to 2000mg is reached by 200mg of extract.

Nettle root extract is used in amounts 500 - 2000mg per dose. The daily dose of Nettle root extract is equivalent to 2000mg Nettle root.

**LYCOPENE-** is the red pigment found in tomatoes, and the most powerful antioxidant in the carotenoid family. It is an oxidant, thus protecting the prostate cells from oxidative stress which can cause chronic inflammation. It plays an important role in cardiovascular health supporting a healthy blood flow and improving capillary circulation.

It has a dual effect of protecting the prostate cells from oxidative damage while its improved capillary blood flow effect enables and increases the effects of all the ingredients in the current composition through enhanced delivery at cellular level.

As Lycopene may increase the risk of bleeding in people taking anti-coagulants, its use should be in adequate amounts.

Lycopene is used in in the current composition in a range of 5-20mg per dose. The daily dose of Lycopene is 20mg.

**SELENIUM** is a micronutrient that humans cannot produce and need in daily amounts for health maintenance. In the current composition Selenium has a protective effect at cellular level and cancer prevention potential. It exerts its protective actions through multiple mechanisms that involve antioxidant activities protecting the prostate cells from oxidative damage and inhibition of DNA damage, inducing apoptosis, inhibiting angiogenesis and invasion. There is a known connection between selenium supplementation and chemo-protective anti-cancer activity. This biological phenomenon may be due to the ability of selenium to instigate cellular apoptosis. However, Selenium is toxic at doses over 400mcg, causing peripheral neuropathy, gastric symptoms, liver, kidney and heart problems, and alopecia. Due to its toxicity, selenium cannot be used as a single ingredient (a dose of 750 mg - represents 750,000 mcg - would be lethal) it can only be used in very small amounts, and in order to benefit from its protective effects against carcinogenesis is used in the current formulation in very small amounts for the synergistic effect it delivers in combination with the other ingredients.

Selenium is used in the range of 40-160mcg per dose. The daily dose of selenium is 160mg.

In some embodiments the Selenium in the form of one of L-Selenomethionine ,Sodium selenate (Na₂SeO₄) or Sodium selenite (Na₂SeO₃). In further embodiments the Selenium is in the form of Sodium selenate or Sodium selenite.

Generally the inventive composition includes Pumpkin seed extract, Nettle root extract (Urtica dioica); Berberine; Emblica officinalis (Amla berry); Ganoderma Lucidum (Reishi mushroom); Lycopene; and Selenium.

More particularly in a first embodiment each dose of the composition includes equivalent to 1000-4000mg of Pumpkin seed (extract); equivalent to 500-2000mg of Nettle root (extract) (Urtica doica); 50-200mg of Berberine Hydrochloride 90%; 50-200mg of Emblica officinalis (Amla berry); 50-200mg of Ganoderma Lucidum (Reishi mushroom); 5-20mg of Lycopene; and 40-160mcg of one of Selenium in the form of L-Selenomethionine or Sodium selenate.

In another embodiment of the present invention the composition is in the form of a powder. Each dose of the composition includes 1000-4000mg of Pumpkin seed extract; 500-2000mg of Nettle root extract (Urtica dioica); 50-200mg of Berberine Hydrochloride 90%; 50-200mg of Emblica officinalis (Amla berry); 50-200mg of Ganoderma Lucidum (Reishi mushroom); 5-20mg of Lycopene; and 40-160mcg of Selenium.

In yet another embodiment of the present invention the composition is in the form of a liquid or gel, each dose having the equivalent of between 1000-4000mg Pumpkin seed extract; the equivalent of between 500-2000mg of Nettle root extract (Urtica dioica); 50-200mg of Berberine Hydrochloride 90%; 50-200mg of Emblica officinalis (Amla berry); 50-200mg of Ganoderma Lucidum (Reishi mushroom);5 -20mg Lycopene; and 40-160mcg Selenium.

In still another embodiment of the present invention the composition is in the form of a tablet Each tablet of the composition includes 1000-4000mg of Pumpkin seed extract; 500-2000mg of Nettle root extract (Urtica dioica); 50-200mg of Berberine Hydrochloride 90%; 50-200mg of Emblica officinalis (Amla berry); 50-200mg of Ganoderma Lucidum (Reishi mushroom); 5-20mg of Lycopene; and 40-160mcg of Selenium.

In a preferred embodiment a capsule or tablet format comprises per tablet/capsule dose:
Pumpkin seed extract equivalent to: 1000mg
Nettle root extract (Urtica dioica) equivalent to: 50mg
Berberine Hydrochloride 90%: 50mg
Emblica Officinalis (Amla berry): 50mg
Ganoderma Lucidum ( Reishi mushroom): 50mg
Lycopene: mg
Selenium: 40mcg.

2-4 capsules/tablets can be administered per day to achieve the preferred daily dose.

The preferred daily dose of the present composition is:
Pumpkin seed extract equivalent to 4000mg of Pumpkin seed;
Nettle root extract (Urtica dioica) equivalent to 2000mg of Nettle root;
Berberine Hydrochloride 200-300mg;
Emblica Officinalis (Amla berry) 200-300mg;
Ganoderma Lucidum (Reishi mushroom) 200-300mg;
Lycopene 20mg; and
Selenium 160mcg.

The preferred daily dose can be provided as a single dose in the form of a gel, a powder or a liquid. Or when the preferred daily dose is provided in capsule or tablet format this can be achieved by 2-4 capsules or tablets depending on their size.

The compositions in the form of liquid or gel can be in a suitable vehicle such as Polyethylene glycol ("PEG") or coconut oil with added flavourings and sweeteners like stevia.

The compositions described herein may consist of the above listed ingredients and optionally also including pharmaceutically acceptable excipient, bulking agents, in the form of a vegetarian capsule for example a Hydroxypropyl Methylcellulose capsule.

The compositions described herein may consist of the above listed ingredients in the form of a tablet, and optionally also including pharmaceutically acceptable excipient, bulking agents, glazing agents and coating agents.

A further aspect of the present invention is the composition for use in combating and reliving the symptoms of prostate enlargement, the composition for use as prevention of prostate cancer. The compositions of the present invention may be used daily.

As a result of the synergies amongst the ingredients, the proposed composition taken daily acts through different mechanisms on multiple cellular pathways, having an inhibitory effect on prostate tumor initiation and development as demonstrated by laboratory experiment described above (See Example 1).

Due to the way ingredients work synergistically, the current composition was also shown to improve the urinary symptoms triggered by an enlarged prostate as demonstrated by the pilot study shown above (See Example 2). The unexpected effect observed after 6 months of use is an improvement in sexual function, both in terms of erectile function as well as frequency of sexual activity, as reported by all the subjects.

### ADMINISTRATION PROTOCOL

Preferred administration is 2-4 capsules/tablets depending on the tablet/capsule size, or one gel sachet daily taken with food.

**DOSE** - the composition could take the form of a powder in a capsule wherein the capsule is a vegetarian capsule of Hydroxypropyl Methylcellulose. The composition can also be administered in the form tablet, liquid or gel in a sachet. The gel embodiment can include Phosphatidylcholine, flavorings, natural sweeteners like for example, stevia and preservatives. The capsule and tablet embodiments may also include pharmaceutically acceptable flowing agents, anti-caking agents and coating agents.

### Examples

### Example 1

Objective of the experiment was to examine the effect of the current composition on prostate cancer cells PC-3 cell line viability and apoptosis.

### Materials

PC-3 Prostate Cancer Cells (human prostate adenocarcinoma derived cell line ATCC CRL-1435) were used.

To prepare growth medium for PC-3 cell line, Nutrient Mixture F-12(Ham's) with L-glutamine was supplemented with 10% v/v FBS, 1% v/v sodium pyruvate and 1% v/v Penicillin-Streptomycin. The medium was stored at 4 degrees Celsius until use. The composition was dissolved in Dulbecco's phosphate-buffered saline ("DPBS").

### Procedure

PC-3 cell lines were cultured in T75 flasks in full growth medium for 3 passages at 370 C in 5% CO2. 10,000 cells/well were seeded in two 96-well plates.

### Methods

Plates were incubated overnight at 37°C in 5% CO2 to allow attachment.

Fresh growth medium containing 10mg/ml of the composition on was applied on PC-3 cells.

One plate was incubated for 24 hours and the other plate was incubated for 48 hours at 370 C in 5% CO2

All the treatments were done in quadruplicate.

Cells used as control were treated with medium containing 5% DPBS as vehicle control.

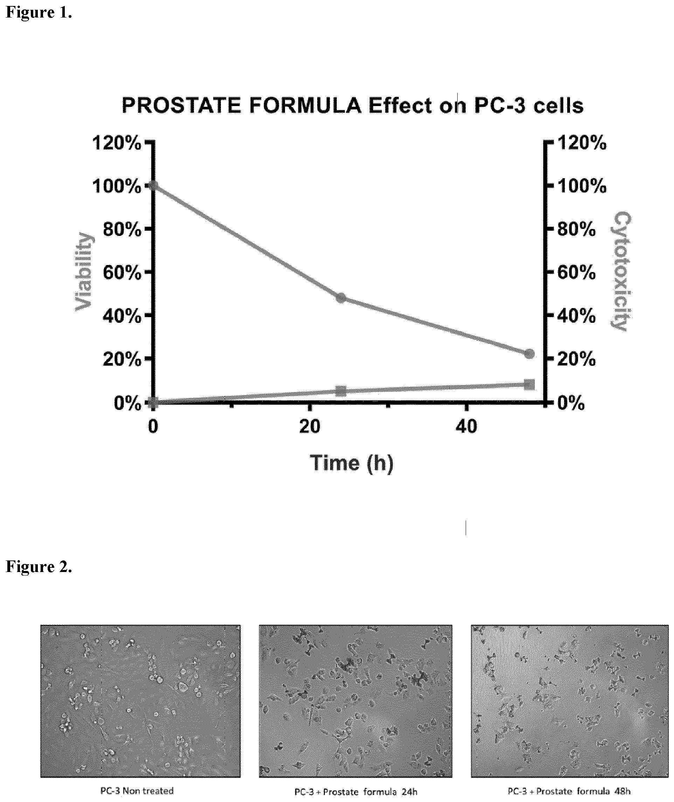

Results: Reference is made to Fig. 1 showing the effect of the composition on cell viability and cytotoxicity (apoptosis) following 24 and 48 hours after application of the current composition.

References is also made to field images of PC-3 cells in Figure 2, before application of current tested composition (non treated) as well as 24 and 48 hours after the application.

Reference is made to Figure 3 which shows the effect of the individual ingredients: pumpkin seed extract, Nettle root (Urtica dioica), Reishi, Amla berry and Berberine; a control and the current composition on cell viability and cytotoxicity (apoptosis) following 48 hours after application. Figure 3 demonstrates the synergistic anti-cancer effect of the current composition in comparison to the ingredients alone.

### Example 2

The current composition was also shown to improve the urinary symptoms triggered by an enlarged prostate as demonstrated by the pilot study shown below. This pilot study was done using the International Prostate Symptom Score (IPSS) developed by the American Urology Association and adopted as global standard. IPSS is the official worldwide assessment for prostate enlargement (BPH) based on answers to seven questions concerning urinary symptoms and one question concerning quality of life.

Five male subjects aged 60-66 completed the questionnaire at the start of the study and after 3 months of daily administration of the proposed prostate health composition. Initial scoring shown below:
Subject 1 Moderate Score 16
Subject 2 Moderate Score 19
Subject 3 Moderate Score 18
Subject 4 Severe Score 28
Subject 5 Severe Score 31

The subjects started taking a daily dose of the Prostate health composition, taken with food. After 3 months they were asked to redo the questionnaire. All subjects indicated a significant improvement in urinary symptoms and comfort, for example:
Frequency- was improved by an average of 40%.
Initial average score was 4. After 3 months the average score was 2.4.
Nocturia - number of night bathroom runs, was improved by an average of 40%.
Initial average score 4.6. After 3 months the average score was 2.4.
Straining - was improved by an average of 25%.
Initial average score was 4. After 3 months the average score was 3.
Urgency - was improved by an average of 30%
Initial average score 3.4. After 3 months the average score was 2.4.

Quality of life due to urinary symptoms score improved from an average of 4 to an average of 2 representing 50% improvement.

Example 2 demonstrates that the present composition improves the urinary symptoms triggered by an enlarged prostate and the quality of life of the subject.

A follow - up of subjects after 6 months highlighted an unexpected effect of the prostate composition. All the subjects reported an improvement in sexual activity. This includes both a significant improvement in erectile function, that previously required medication, as well as duration and frequency of sexual activity which increased compared to the period before starting.

## Claims

1. A composition comprising:
Pumpkin seed extract;
Nettle root extract (Urtica dioica);
Berberine;
Emblica officinalis (Amla berry);
Ganoderma Lucidum (Reishi mushroom);
Lycopene; and
Selenium.

2. A composition according to claim 1 wherein the composition comprises:
the Pumpkin seed extract is in an amount equivalent to 1000-4000mg Pumpkin seed per dose;
the Nettle root extract (Urtica dioica) is in an amount equivalent to 500-2000mg Nettle root per dose;
the Berberine in the form of Berberine Hydrochloride 90% is in an amount of 50-300mg per dose;
the Emblica officinalis (Amla berry) is in an amount of 50-300mg per dose;
the Ganoderma Lucidum (Reishi mushroom) is in an amount of 50-300mg per dose;
the Lycopene is in an amount of 5-20mg per dose; and
the Selenium is in an amount of 40-160mcg.

3. A composition according to claim 1 or 2 wherein the composition comprises:
Pumpkin seed extract equivalent to 4000mg of Pumpkin seed per daily dose;
Nettle root extract (Urtica dioica) equivalent to 2000mg of Nettle root per daily dose;
Berberine in the form of Berberine Hydrochloride 200-300mg per daily dose;
Emblica Officinalis (Amla berry) 200-300mg per daily dose;
Ganoderma Lucidum (Reishi mushroom) 200-300mg per daily dose;
Lycopene 20mg per daily dose; and
Selenium 160mcg per daily dose.

4. A composition according to any one of claims 1-3, wherein the composition is in the form of one of a powder, liquid or a gel.

5. The composition of any one of claims 1-4, wherein the composition is in the form of a liquid, gel or powder comprising:
400mg of 10:1 Pumpkin seed extract equivalent to 4000mg Pumpkin seed;
200mg of 10:1 Nettle root (Urtica dioica) doica) extract equivalent to 2000mg Nettle root;
200-300mg of Berberine Hydrochloride;
200-300mg of Emblica Officinalis (Amla berry);
200-300mg of Ganoderma lucidum (Reishi mushroom);
20mg of Lycopene; and
160mcg of Selenium.

6. The composition of claims 1 or 2, wherein the composition is in the form of a tablet or capsule comprising:
Pumpkin seed extract equivalent to 1000-2000mg Pumpkin seed;
Nettle root extract (Urtica dioica) equivalent to 500-1000mg Nettle root;
50-150mg of Berberine Hydrochloride 90%;
50-150mg of Emblica officinalis (Amla berry);
50-150mg of Ganoderma Lucidum (Reishi mushroom);
5-10mg of Lycopene; and
40-80mcg of Selenium.

7. The composition according to any one of claims 1-3 or 6, wherein composition is the form of a powder in a capsule optionally wherein the capsule is a vegetarian capsule of Hydroxypropyl Methylcellulose.

8. The composition of any one of claims 1-7, further comprising one or more of pharmaceutically acceptable diluents, lubricants, glazing and coating agents.

9. The composition according to any of the preceding claims wherein the Selenium is in the form of one of L-Selenomethionine, sodium selenate or sodium selenite.

10. A composition for use in preventing prostate cancer and protecting the prostate cells from malignant changes in a subject or mitigating the urinary symptoms of an enlarged prostate comprising:
Pumpkin seed extract;
Nettle root extract (Urtica dioica);
Berberine Hydrochloride;
Emblica officinalis (Amla berry);
Ganoderma Lucidum (Reishi mushroom);
Lycopene; and
Selenium; and
optionally one or more of pharmaceutically acceptable diluents, lubricants, glazing and
coating agents.

11. The composition according to any one of claims 1-9, for use in significantly lowering the risk of prostate cancer and protecting the prostate cells from malignant changes. in a subject

12. A composition according to any one of claims 1-9, for use in preventing prostate cancer and protecting the prostate cells from malignant changes in a subject with daily administration wherein the dose comprises:
Pumpkin seed extract is in an amount of 4000mg Pumpkin seed per daily dose;
Nettle root extract (Urtica dioica) is in an amount of 2000mg Nettle root per daily dose;
Berberine Hydrochloride 90% is in an amount of 200-300mg per daily dose;
Emblica officinalis (Amla berry) is in an amount of 200-300mg per daily dose;
Ganoderma Lucidum (Reishi mushroom) is in an amount of 200-300mg per daily dose;
Lycopene is in an amount of 20mg per daily dose; and
Selenium is in an amount of 160mcg in the form of one of L-Selenomethionine or Sodium selenate per daily dose.

13. A composition according to any one of the claims 1-9, for use in mitigating the urinary symptoms of an enlarged prostate.

14. A composition according to any one of claims 1-9, for use in treating urinary symptoms triggered by an enlarged prostate optionally wherein the urinary symptoms are selected from frequency, nocturia, straining and urgency of urination.

15. A composition according to any one of claims 1-9, for use in treating erectile dysfunction or for non-therapeutic use for improving sexual function.

## Patentansprüche

1. Zusammensetzung, umfassend:
Kürbiskern-Extrakt;
Brennnesselwurzel-Extrakt (Urtica dioica);
Berberin;
Embilica officinalis (Amla-Beere);
Ganoderma lucidum (Reishi-Pilz);
Lycopin; und
Selen.

2. Zusammensetzung nach Anspruch 1, wobei die Zusammensetzung Folgendes umfasst:
den Kürbiskern-Extrakt in einer Menge, die 1000 bis 4000 mg Kürbiskernen pro Dosis entspricht;
den Brennnesselwurzel-Extrakt (Urtica dioica) in einer Menge, die 500 bis 2000 mg Brennnesselwurzel pro Dosis entspricht;
Berberin in Form von Berberinhydrochlorid 90 % in einer Menge von 50 bis 300 mg pro Dosis;
Embilica officinalis (Amla-Beere) in einer Menge von 50 bis 300 mg pro Dosis;
Ganoderma lucidum (Reishi-Pilz) in einer Menge von 50 bis 300 mg pro Dosis;
Lycopin in einer Menge von 5 bis 20 mg pro Dosis; und
Selen in einer Menge von 40 bis 160 µg.

3. Zusammensetzung nach Anspruch 1 oder 2, wobei die Zusammensetzung Folgendes umfasst:
Kürbiskern-Extrakt, entsprechend 4000 mg Kürbiskerne pro Tagesdosis;
Brennnesselwurzel-Extrakt (Urtica dioica), entsprechend 2000 mg Brennnesselwurzel pro Tagesdosis;
Berberin in Form von Berberinhydrochlorid 200 bis 300 mg pro Tagesdosis;
Embilica officinalis (Amla-Beere) 200 bis 300 mg pro Tagesdosis;
Ganoderma lucidum (Reishi-Pilz) 200 bis 300 mg pro Tagesdosis;
Lycopin 20 mg pro Tagesdosis; und
Selen 160 µg pro Tagesdosis.

4. Zusammensetzung nach einem der Ansprüche 1 bis 3, wobei die Zusammensetzung in Form eines aus einem Pulver, einer Flüssigkeit oder einem Gel vorliegt.

5. Zusammensetzung nach einem der Ansprüche 1 bis 4, wobei die Zusammensetzung in Form einer Flüssigkeit, eines Gels oder eines Pulvers vorliegt, die/das Folgendes umfasst:
400 mg von 10:1 Kürbiskern-Extrakt, entsprechend 4000 mg Kürbiskernen;
200 mg von 10:1 Brennnesselwurzel-Extrakt (Urtica dioica), entsprechend 2000 mg Brennnesselwurzel;
200 bis 300 mg Berberinhydrochlorid;
200 bis 300 mg Embilica officinalis (Amla-Beere);
200 bis 300 mg Ganoderma lucidum (Reishi-Pilz);
20 mg Lycopin; und
160 µg Selen.

6. Zusammensetzung nach Anspruch 1 oder 2, wobei die Zusammensetzung in Form einer Tablette oder Kapsel vorliegt, die Folgendes umfasst:
Kürbiskern-Extrakt, entsprechend 1000 bis 2000 mg Kürbiskernen;
Brennnesselwurzel-Extrakt (Urtica dioica), entsprechend 500 bis 1000 mg Brennnesselwurzel;
50 bis 150 mg Berberinhydrochlorid 90 %;
50 bis 150 mg Embilica officinalis (Amla-Beere);
50 bis 150 mg Ganoderma lucidum (Reishi-Pilz);
5 bis 10 mg Lycopin; und
40 bis 80 µg Selen.

7. Zusammensetzung nach einem der Ansprüche 1 bis 3 oder 6, wobei die Zusammensetzung in Form eines Pulvers in einer Kapsel vorliegt, wobei die Kapsel gegebenenfalls eine vegetarische Kapsel aus Hydroxypropylmethylcellulose ist.

8. Zusammensetzung nach einem der Ansprüche 1 bis 7, die weiters eines oder mehrere aus pharmazeutisch annehmbaren Verdünnungsmitteln, Gleitmitteln, Überzugsmitteln und Beschichtungsmitteln umfasst.

9. Zusammensetzung nach einem der vorangegangenen Ansprüche, wobei das Selen in Form eines aus L-Selenomethionin, Natriumselenat oder Natriumselenit vorliegt.

10. Zusammensetzung zur Verwendung bei der Prävention von Prostatakrebs und zum Schutz von Prostatazellen gegen maligne Veränderungen in einem Individuum oder zum Lindern der Harnwegssymptome einer vergrößerten Prostata, umfassend:
Kürbiskern-Extrakt;
Brennnesselwurzel-Extrakt (Urtica dioica);
Berberinhydrochlorid;
Embilica officinalis (Amla-Beere);
Ganoderma lucidum (Reishi-Pilz);
Lycopin; und
Selen; und
gegebenenfalls eines oder mehrere aus pharmazeutisch annehmbaren Verdünnungsmitteln, Gleitmitteln, Überzugsmitteln und Beschichtungsmitteln.

11. Zusammensetzung nach einem der Ansprüche 1 bis 9 zur Verwendung zum signifikanten Verringern des Risikos für Prostatakrebs und zum Schutz der Prostatazellen vor malignen Veränderungen in einem Individuum.

12. Zusammensetzung nach einem der Ansprüche 1 bis 9 zur Verwendung bei der Prävention von Prostatakrebs und zum Schutz der Prostatazellen vor malignen Veränderungen in einem Individuum bei täglicher Verabreichung, wobei die Dosis Folgendes umfasst:
Kürbiskern-Extrakt in einer Menge von 4000 mg Kürbiskernen pro Tagesdosis;
Brennnesselwurzel-Extrakt (Urtica dioica) in einer Menge von 2000 mg Brennnesselwurzel pro Tagesdosis;
Berberinhydrochlorid 90 % in einer Menge von 200 bis 300 mg pro Tagesdosis;
Embilica officinalis (Amla-Beere) in einer Menge von 200 bis 300 mg pro Tagesdosis;
Ganoderma lucidum (Reishi-Pilz) in einer Menge von 200 bis 300 mg pro Tagesdosis;
Lycopin in einer Menge von 20 mg pro Tagesdosis; und
Selen in einer Menge von 160 µg in der Form eines aus L-Selenomethionin oder Natriumselenat pro Tagesdosis.

13. Zusammensetzung nach einem der Ansprüche 1 bis 9 zur Verwendung bei der Linderung von Harnwegssymptomen einer vergrößerten Prostata.

14. Zusammensetzung nach einem der Ansprüche 1 bis 9 zur Verwendung bei der Behandlung von Harnwegssymptomen, die durch eine vergrößerte Prostata ausgelöst werden, wobei die Harnwegssymptome gegebenenfalls aus Häufigkeit, Nykturie, schmerzhaftem Urinieren und Harndrang ausgewählt sind.

15. Zusammensetzung nach einem der Ansprüche 1 bis 9 zur Verwendung bei der Behandlung erektiler Dysfunktion oder zur nichttherapeutischen Verwendung zur Verbesserung der Sexualfunktion.

## Revendications

1. Composition comprenant :
de l'extrait de graines de citrouille ;
de l'extrait de racine d'ortie (Urtica dioica) ;
de la berbérine ;
de l'Emblica officinalis (baie d'Amla) ;
du Ganoderma Lucidum (champignon Reishi) ;
du lycopène ; et
du sélénium.

2. Composition selon la revendication 1, dans laquelle la composition comprend :
l'extrait de graines de citrouille en une quantité qui équivaut à 1 000 à 4 000 mg de graines de citrouille par dose ;
l'extrait de racine d'ortie (Urtica dioica) en une quantité qui équivaut à 500 à 2 000 mg de racine d'ortie par dose ;
la berbérine sous la forme de chlorhydrate de berbérine à 90 % en une quantité de 50 à 300 mg par dose ;
l'Emblica officinalis (baie d'Amla) en une quantité de 50 à 300 mg par dose ;
du Ganoderma Lucidum (champignon Reishi) en une quantité de 50 à 300 mg par dose ;
du lycopène en une quantité de 5 à 20 mg par dose ; et
du sélénium en une quantité de 40 à 160 mcg.

3. Composition selon la revendication 1 ou 2, dans laquelle la composition comprend :
de l'extrait de graines de citrouille qui équivaut à 4 000 mg de graines de citrouille par dose quotidienne ;
de l'extrait de racine d'ortie (Urtica dioica) qui équivaut à 2 000 mg de racine d'ortie par dose quotidienne ;
de la berbérine sous forme de chlorhydrate de berbérine, de 200 à 300 mg par dose quotidienne ;
de l'Emblica Officinalis (baie d'Amla), de 200 à 300 mg par dose quotidienne ;
du Ganoderma Lucidum (champignon Reishi), de 200 à 300 mg par dose quotidienne ;
du lycopène, 20 mg par dose quotidienne ; et
du sélénium, 160 mcg par dose quotidienne.

4. Composition selon l'une quelconque des revendications 1 à 3, dans laquelle la composition est sous la forme d'une poudre, d'un liquide ou d'un gel.

5. Composition selon l'une quelconque des revendications 1 à 4, dans laquelle la composition est sous la forme d'un liquide, d'un gel ou d'une poudre comprenant :
400 mg d'extrait de graines de citrouille à 10:1, ce qui équivaut à 4 000 mg de graines de citrouille ;
200 mg d'extrait de racine d'ortie (Urtica dioica) à 10:1, ce qui équivaut à 2 000 mg de racine d'ortie ;
de 200 à 300 mg de chlorhydrate de berbérine ;
de 200 à 300 mg d'Emblica Officinalis (baie d'Amla) ;
de 200 à 300 mg de Ganoderma lucidum (champignon Reishi) ;
20 mg de lycopène ; et
160 mcg de sélénium.

6. Composition selon les revendications 1 ou 2, dans laquelle la composition est sous la forme d'un comprimé ou d'une capsule comprenant :
de l'extrait de graines de citrouille qui équivaut à 1 000 à 2 000 mg de graines de citrouille ;
de l'extrait de racine d'ortie (Urtica dioica) qui équivaut à 500 à 1 000 mg de racine d'ortie ;
de 50 à 150 mg de chlorhydrate de berbérine à 90 % ;
de 50 à 150 mg d'Emblica officinalis (baie d'Amla) ;
de 50 à 150 mg de Ganoderma Lucidum (champignon Reishi) ;
de 5 à 10 mg de lycopène ; et
de 40 à 80 mcg de sélénium.

7. Composition selon l'une quelconque des revendications 1 à 3 ou 6, dans laquelle la composition est la forme d'une poudre dans une capsule, facultativement dans laquelle la capsule est une capsule d'origine végétale d'hydroxypropylméthylcellulose.

8. Composition selon l'une quelconque des revendications 1 à 7, comprenant en outre un ou plusieurs diluants, lubrifiants, agents d'enrobage et de revêtement pharmaceutiquement acceptables.

9. Composition selon l'une quelconque des revendications précédentes, dans laquelle le sélénium est sous la forme d'un parmi la L-sélénométhionine, le sélénate de sodium ou le sélénite de sodium.

10. Composition à utiliser pour prévenir le cancer de la prostate et protéger les cellules de la prostate à l'encontre de changements malins chez un sujet ou atténuer les symptômes urinaires d'une hypertrophie de la prostate, comprenant :
de l'extrait de graines de citrouille ;
de l'extrait de racine d'ortie (Urtica dioica) ;
du chlorhydrate de berbérine ;
de l'Emblica officinalis (baie d'Amla) ;
du Ganoderma Lucidum (champignon Reishi) ;
du lycopène ; et
du sélénium ; et
facultativement un ou plusieurs diluants, lubrifiants, agents d'enrobage et de revêtement pharmaceutiquement acceptables.

11. Composition selon l'une quelconque des revendications 1 à 9, à utiliser pour réduire de manière significative le risque de cancer de la prostate et protéger les cellules de la prostate à l'encontre de changements malins chez un sujet.

12. Composition selon l'une quelconque des revendications 1 à 9, à utiliser dans la prévention du cancer de la prostate et la protection des cellules de la prostate à l'encontre de changements malins chez un sujet avec une administration quotidienne, dans laquelle la dose comprend :
de l'extrait de graines de citrouille en une quantité de 4 000 mg de graines de citrouille par dose quotidienne ;
de l'extrait de racine d'ortie (Urtica dioica) en une quantité de 2 000 mg de racine d'ortie par dose quotidienne ;
du chlorhydrate de berbérine à 90 % en une quantité de 200 à 300 mg par dose quotidienne ;
de l'Emblica officinalis (baie d'Amla) en une quantité de 200 à 300 mg par dose quotidienne ;
du Ganoderma Lucidum (champignon Reishi) en une quantité de 200 à 300 mg par dose quotidienne ;
du lycopène en une quantité de 20 mg par dose quotidienne ; et
du sélénium en une quantité de 160 mcg sous la forme de L-sélénométhionine ou de sélénate de sodium par dose quotidienne.

13. Composition selon l'une quelconque des revendications 1 à 9**,** à utiliser pour atténuer les symptômes urinaires d'une hypertrophie de la prostate.

14. Composition selon l'une quelconque des revendications 1 à 9, à utiliser dans le traitement des symptômes urinaires déclenchés par une hypertrophie de la prostate, facultativement dans laquelle les symptômes urinaires sont choisis parmi la fréquence, la nycturie, la fatigue et l'urgence de la miction.

15. Composition selon l'une quelconque des revendications 1 à 9**,** à utiliser dans le traitement de la dysfonction érectile ou à usage non thérapeutique pour améliorer la fonction sexuelle.
